(19) Europäisches Patentamt
European Patent Office
Office européen des brevets

(11) Veröffentlichungsnummer: **0 087 558**
**A1**

# (12) EUROPÄISCHE PATENTANMELDUNG

(21) Anmeldenummer: 83100205.0

(22) Anmeldetag: 12.01.83

(51) Int. Cl.$^3$: **C 07 D 339/04**

(30) Priorität: 21.01.82 DE 3201761

(43) Veröffentlichungstag der Anmeldung: 07.09.83 Patentblatt 83/36

(84) Benannte Vertragsstaaten: BE CH DE FR GB IT LI NL

(71) Anmelder: CASSELLA Aktiengesellschaft
Hanauer Landstrasse 526
D-6000 Frankfurt am Main 61(DE)

(72) Erfinder: Müller, Rolf, Dr.
Dornbachweg 3
D-6367 Karben-4(DE)

(72) Erfinder: Wille, Herbert, Dr.
Hünfelder Strasse 16
D-6000 Frankfurt am Main 61(DE)

(74) Vertreter: Urbach, Hans-Georg, Dr. et al,
Hanauer Landstrasse 526
D-6000 Frankfurt am Main 61(DE)

## (54) Verfahren zur Herstellung von 4.5-Dichlor-1.2-dithiacyclopentenon-(3).

(57) Bei dem Verfahren zur Herstellung von 4.5-Dichlor-1.2-dithia-cyclopentenon-(3) wird Hexachlorpropen mit Schwefel bei erhöhter Temperatur umgesetzt und während der Reaktion Wasser zugefügt.

EP 0 087 558 A1

## Verfahren zur Herstellung von 4.5-Dichlor-1.2-dithia-cyclopentenon-(3)

Die Erfindung betrifft ein Verfahren zur Herstellung von 4.5-Dichlor-1.2-dithia-cyclopentenon-(3), auch 5-Oxo-3.4-dichlor-1.2-dithiol genannt, der Formel I

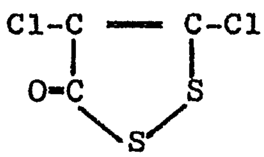

(I)

das auch in technischem Maßstab durchgeführt werden kann.

4.5-Dichlor-1.2-dithia-cyclopentenon-(3) wird z.B. zur Schleimpilz-Bekämpfung in der Papierindustrie eingesetzt, vgl. DE-PS 29 13 593. Zur Herstellung der Verbindung der Formel I sind zwei Verfahren bekannt, nämlich 1. die Umsetzung von Hexachlorpropen mit Schwefel oder Schwefelchloriden in Gegenwart eines Friedel-Crafts-Katalysators (DE-PS 11 02 174 und Friedrich Boberg: Ann.Chem.679 (1964), 109 bis 118) und 2. die Umsetzung von Hexachlorpropen mit Schwefel bei Temperaturen oberhalb von 160°C (DE-PS 11 28 432 und Friedrich Boberg loc.cit.). Bei dem zuerst genannten Verfahren setzt die Reaktion zwischen Hexachlorpropen, Schwefel und Aluminiumchlorid in einem engen Temperaturbereich, etwa bei 46°C sehr heftig ein, so daß die entstehende Wärmemenge bei größeren Ansätzen nicht mehr abzuführen ist. Ferner erstarrt das Reaktionsgemisch kurze Zeit nach Einsetzen der Reaktion vollständig, und der Rührer bleibt stehen. Auch ein großer Überschuß an Hexachlorpropen kann diesem Übelstand nicht abhelfen. Übliche Lösungsmittel, wie Dichlorbenzol oder Nitrobenzol, werden angegriffen. Außerdem bildet sich immer etwas Hexachlorethan, das aufgrund seines leichten Sublimierens bei Ansätzen in technischem Maßstab nicht abgetrennt werden kann. Aus diesen Gründen ist eine störungsfreie technische Produktion nach dem zuerst genannten Verfahren

nicht möglich.

Bei beiden Verfahren wird das gewünschte Endprodukt durch Hydrolyse der zunächst entstehenden Zwischenprodukte erhalten. Bei dem zuerst genannten Verfahren ist dies das 3.4.5-Trichlor-1.2-dithiolium-$AlCl_4$-Addukt und beim zweiten Verfahren das 3.4.5-Trichlor-1.2-dithioliumchlorid. Beim zweiten Verfahren wird das kristalline Zwischenprodukt zur Entfernung von gebildeten, anhaftenden Schwefelchloriden abgesaugt und mit Benzol und anschließend mit Schwefelkohlenstoff gewaschen. Dies ist in technischem Maßstab mit Schwierigkeiten verbunden, so daß sich auch dieses Verfahren für eine Herstellung der Verbindung der Formel I in technischem Maßstab nicht eignet. Falls die Schwefelchloride vor der Hydrolyse des 3.4.5-Trichlor-1.2-dithioliumchlorids nicht vollständig entfernt werden, entstehen bei der Hydrolyse gummiartige Produkte, die eine nachfolgende Destillation des 4.5-Dichlor-1.2-dithiacyclopentenons-(3) unmöglich machen.
Bei beiden bekannten Verfahren werden größere Mengen Schwefelchloride, insbesondere Schwefeldichlorid und Dischwefeldichlorid, gebildet, was aus sicherheitstechnischen Gründen beträchtliche Probleme aufwirft. Auch bereitet die spätere Vernichtung der Schwefelchloride zusätzliche Schwierigkeiten. Nach Friedrich Boberg loc.cit., Seite 110, ist jedoch die Anwesenheit von Dischwefeldichlorid bei der Umsetzung von Hexachlorpropen und Schwefel von Bedeutung, denn durch Abdestillieren des $S_2Cl_2$ während der Umsetzung wird eine schlechtere Ausbeute erhalten.

Es wurde nun überraschenderweise gefunden, daß eine Herstellung von 4.5-Dichlor-1.2-dithia-cyclopentenon-(3) auch in technischem Maßstab möglich ist, wenn die bei der Umsetzung von Hexachlorpropen und Schwefel entstehenden Schwefelchloride in dem Maße, wie sie entstehen, durch Zusatz von Wasser hydrolysiert werden. Die Erfindung betrifft daher ein Verfahren zur Herstellung von 4.5-Dichlor-1.2-dithia-cyclopentenon-(3) durch Umsetzung von Hexachlorpropen mit Schwefel bei erhöhten Temperaturen, das dadurch gekennzeichnet ist, daß während der Reaktion Wasser zugefügt wird. Zweckmäßigerweise wird das Wasser kontinuierlich während der gesamten Reaktionszeit zugefügt. Das Wasser kann in flüssiger oder dampfförmiger Form zugefügt werden.

Die Reaktionstemperatur ist etwa von 150°C bis zum Siedepunkt des Hexachlorpropens (210°C) variierbar. Aus Gründen der Reaktionsgeschwindigkeit wird sie jedoch vorteilhaft zwischen 155°C und 185°C, vorzugsweise zwischen 157° und 175°C, gehalten; eine relativ genaue Temperaturführung erleichtert die Reaktionsführung. Das molare Verhältnis zwischen Hexachlorpropen, Schwefel und Wasser kann in weiten Grenzen variiert werden, da das nicht umgesetzte Hexachlorpropen wieder zurückgewonnen wird. Zweckmäßigerweise wird in jedem Reaktionsstadium soviel Wasser zugefügt, wie zur Hydrolyse der primär gebildeten Reaktionsprodukte erforderlich ist, von denen die Schwefelchloride leicht durch ihre gelbe Farbe im Rückfluß erkannt werden können. Normalerweise beträgt , bezogen auf tatsächlich umgesetztes Hexachlorpropen, das Molverhältnis Hexachlorpropen : Schwefel : Wasser = 1 : (2,5 bis 3,0) : (2,0 bis 2,5). Mehr Wasser oder mehr Schwefel anzuwenden, ist nicht erforderlich. Wird mehr Schwefel eingesetzt, dann verbleibt Schwefel im Destillationsrückstand, was aus ökologischen Gründen unerwünscht ist. Dagegen ist es vorteilhaft, Hexachlorpropen im Überschuß einzusetzen, dabei ist ein Überschuß von 5 bis 25 Gew.% in der Regel ausreichend, so daß sich für die einzusetzenden Reaktionspartner ein Molverhältnis Hexachlorpropen : Schwefel : Wasser = 1 : (2 bis 2,9) : (1,6 bis 2,4) ergibt. Bei richtig bemessenem Wasserzusatz sind bei Reaktionsende keine Schwefelchloride mehr im Reaktionsgemisch vorhanden.

Es kann angenommen werden, daß bei dem erfindungsgemäßen Verfahren bei der Umsetzung zwischen Hexachlorpropen und Schwefel intermediär 3.4.5-Trichlor-1.2-dithiolium-chlorid II

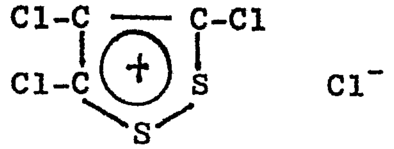

(II)

und Schwefelchloride, insbesondere $S_2Cl_2$, entstehen und daß die intermediär gebildeten Produkte sofort von dem anwesenden Wasser bei den hohen Reaktionstemperaturen hydrolysiert werden, wobei aus dem 3.4.5-Trichlor-1.2-dithiolium-chlorid (II) das 4.5-Dichlor-1.2-dithia-cyclopentenon-(3) (I) und aus den Schwefelchloriden Schwefel, Schwefeldioxid und Chlorwasserstoff entstehen und daß der so gebildete Schwefel von neuem in die Reaktion eintritt. Demnach ergibt sich als Reaktionsablauf für die Gesamtreaktion etwa folgende Summengleichung:

$$Cl_3C\text{-}CCl{=}CCl_2 + 2{,}5S + 2H_2O \longrightarrow I + 1/2\ SO_2 + 4HCl$$

Da das Wasser in einer solchen Menge zugefügt wird, daß das entstandene 3.4.5-Trichlor-1.2-dithioliumchlorid und die Schwefelchloride rasch hydrolysiert werden, sind die während der Reaktion nach dem erfindungsgemäßen Verfahren vorhandenen stationären Konzentrationen an Schwefelchloriden und 3.4.5-Trichlor-1.2-dithioliumchlorid sowie an Wasser gering; in großen Mengen vorhanden sind nur die stabilen Produkte Hexachlorpropen, Schwefel und 4.5-Dichlor-1.2-dithia-cyclopentenon-(3). Die bei der Hydrolyse der Schwefelchloride entstehenden Gase HCl und $SO_2$ können problemlos in Wasser absorbiert werden. Infolge der geringen, im Reaktionsgemisch vorhandenen Mengen an Schwefelchloriden sind besondere sicherheitstechnische Vorkehrungen nicht erforderlich. Die Dauer der Reaktion richtet sich vor allem nach der gewählten Reaktionstemperatur und der dem Reaktionsgemisch zugeführten Wasser- und Wärmemenge und beträgt in der Regel 6 bis 60 Stunden. Die Aufarbeitung des Reaktionsgemisches erfolgt in der Regel durch Destillation, doch kann auch eine Aufarbeitung durch Kristallisation, z.B. aus Petroläther, erfolgen. Das gegebenenfalls im Überschuß eingesetzte Hexachlorpropen wird durch Destillation des Reaktiongsgemisches als Vorlauf wiedergewonnen und kann ohne weitere Reinigung wieder eingesetzt werden.

Nach dem erfindungsgemäßen Verfahren wird das 4.5-Dichlor-1.2-dithia-cyclopentenon-(3) in einer Ausbeute von ca 90% der Theorie und in einer gaschromatographisch bestimmten Reinheit von über 99 % erhalten.

Beispiel 1

84,6 kg (340 Mole) Hexachlorpropen und 27,2 kg (850 Mole) Schwefel werden in einem 125-l-Emaillekessel mit Ölheizung auf eine Innentemperatur von 165°C erhitzt. Anschließend werden im Verlauf von etwa 8 Stunden durch das Bodenventil 12 kg (667 Mole) Wasserdampf eingeblasen. Die Innentemperatur bleibt zwischen 165°C und 172°C, und im Rückflußkühler ist immer das zweiphasige Gemisch aus Hexachlorpropen und Wasser zu sehen, wobei das Hexachlorpropen durch Schwefelchlorid gelb gefärbt ist. Wenn nach etwa 8 Stunden die 12 kg Wasserdampf zugeführt sind, verschwindet im Rücklauf die gelbe Farbe des Schwefelchlorids, und die Reaktion ist beendet. Es wird auf 80 bis 100°C abgekühlt, nochmals kurz Wasserdampf eingeleitet und dann im Vakuum bei 16 mbar destilliert. Man erhält 5,3kg(21,31 Mole) Hexachlorpropen vom Siedepunkt 80 bis 90°C/16 mbar und 54,1 kg 4.5-Dichlor-1.2-dithia-cyclopentenon-(3) mit einer gaschromatographisch bestimmten Reinheit von 99 %. Ausbeute: 89,9 % der Theorie, bezogen auf umgesetztes Hexachlorpropen.

Beispiel 2

In einem 6-l-Vierhalskolben mit Rührer, Thermometer, Glaseinleitungsrohr und Rückflußkühler werden 1600 g (50 Mole) Schwefel und 5480 g (22 Mole) Hexachlorpropen vorgelegt und unter Rühren in einem Ölbad von 170°C erwärmt. Wenn die Innentemperatur 155°C erreicht hat, wird Wasserdampf eingeleitet. Das Tempo der Einleitung wird so bemessen, daß die Innentemperatur bei 155 bis 160°C bleibt. Nach etwa 45 Stunden sind 720 g (40 Mole) Wasserdampf eingeleitet, und die Reaktion ist beendet. Aufarbeitung, wie in Beispiel 1 beschrieben, ergibt eine Ausbeute von 810 g zurückgewonnenem Hexachlorpropen und von 3247 g 4.5-Dichlor-1.2-dithia-cyclopentenon-(3) mit einer gaschromatographisch bestimmten Reinheit von 99 %. Ausbeute: 91,6 %

der Theorie, bezogen auf umgesetztes Hexachlorpropen.

Beispiel 3

Man verfährt wie in Beispiel 2 beschrieben, tropft jedoch im Verlauf der angegebenen Zeit die angegebene Wassermenge in flüssiger Form durch einen Dosiertrichter mit Glasventil zu. Man erhält das Produkt in ähnlicher Reinheit und Ausbeute.

Beispiel 4

Man verfährt wie in Beispiel 1 beschrieben bis zum Beginn der Aufarbeitung. Anschließend wird der geringe Wasserüberschuß im Vakuum abdestilliert, der Kolbeninhalt auf etwa 60°C abgekühlt und anschließend aus Petroläther umkristallisiert. Man erhält das Produkt in einer Ausbeute von etwa 88 % der Theorie und in einer gaschromatographisch bestimmten Reinheit von über 99,5 %. Das nicht umgesetzte Hexachlorpropen wird durch Destillation der Mutterlauge wiedergewonnen.

PATENTANSPRÜCHE

1. Verfahren zur Herstellung von 4.5-Dichlor-1.2-dithiacyclopentenon-(3) durch Umsetzung von Hexachlorpropen mit Schwefel bei erhöhten Temperaturen, dadurch gekennzeichnet, daß während der Reaktion Wasser zugefügt wird.

2. Verfahren nach Anspruch 1, dadurch gekennzeichnet, daß das Wasser kontinuierlich zugefügt wird.

3. Verfahren nach Anspruch 1 oder 2, dadurch gekennzeichnet, daß das Wasser während der gesamten Reaktionszeit zugefügt wird.

4. Verfahren nach einem oder mehreren der Ansprüche 1 bis 3, dadurch gekennzeichnet, daß das Wasser dampfförmig zugefügt wird.

5. Verfahren nach einem oder mehreren der Ansprüche 1 bis 4, dadurch gekennzeichnet, daß das Wasser einer 150 bis 210°C heißen Reaktionsmischung zugefügt wird.

6. Verfahren nach einem oder mehreren der Ansprüche 1 bis 5, dadurch gekennzeichnet, daß das Wasser einer 155 bis 185°C heißen Reaktionsmischung zugefügt wird.

7. Verfahren nach einem oder mehreren der Ansprüche 1 bis 6, dadurch gekennzeichnet, daß das Wasser einer 157 bis 175°C heißen Reaktionsmischung zugefügt wird.

8. Verfahren nach einem oder mehreren der Ansprüche 1 bis 7, dadurch gekennzeichnet, daß in jedem Reaktionsstadium soviel Wasser zugefügt wird, wie zur Hydrolyse der primär gebildeten Reaktionsprodukte erforderlich ist.

9. Verfahren nach einem oder mehreren der Ansprüche 1 bis 8, dadurch gekennzeichnet, daß pro mol Hexachlorpropen 1,6 bis 2,4 mol Wasser zugefügt wird.

Europäisches Patentamt

**EUROPÄISCHER RECHERCHENBERICHT**

0087558
Nummer der Anmeldung

EP 83 10 0205

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | | | C 07 D 339/04 |
| D,A | DE-C-1 128 432 (CHEMISCHE WERKE ALBERT)<br>* Anspruch 1; Beispiel 1 *<br>--- | 1 | |
| D,A | DE-C-1 102 174 (CHEMISCHE WERKE ALBERT)<br>* Anspruch 1; Beispiele 1-8 *<br>--- | 1 | |
| D,A | JUSTUS LIEBIGS ANNALEN DER CHEMIE, Band 679, 1964 Weinheim F. BOBERG "Über 1,2-Dithia-cyclopentene, V. 4,5-Dichlor-1,2-dithia-cyclopentenon-(3)"<br>* Seite 110, 2. Absatz; Formelschema; 3. Absatz; Seite 116, Zeilen 19-30 *<br>--- | 1 | |
| | | | RECHERCHIERTE SACHGEBIETE (Int. Cl. ³) |
| A | DE-B-1 275 068 (SHELL INTERNATIONALE RESEARCH MAATSCHAPPIJ N.V)<br>* Anspruch 1; Spalte 5, Zeilen 12-18 *<br>----- | | C 07 D 339/00 |

Der vorliegende Recherchenbericht wurde für alle Patentansprüche erstellt.

| Recherchenort | Abschlußdatum der Recherche | Prüfer |
|---|---|---|
| BERLIN | 14-04-1983 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82